# EUROPÄISCHE PATENTSCHRIFT

(11) **EP 2 670 379 B1**
(45) Veröffentlichungstag und Bekanntmachung des Hinweises auf die Patenterteilung: **27.06.2018**
(21) Anmeldenummer: 12703001.3
(22) Anmeldetag: 24.01.2012
(51) Int. Cl.: A61K 8/34, A61K 8/86, A61Q 19/10, A61K 8/04

(54) **SELBSTERWÄRMENDES TENSIDHALTIGES REINIGUNGSGEL MIT EINER KOMBINATION AUS NIEDER- UND HOCHMOLEKULAREN PEGS**
SELF-HEATING, SURFACTANT-CONTAINING CLEANSING GEL FEATURING A COMBINATION OF LOW AND HIGH MOLECULAR MASS PEGS
GEL NETTOYANT CONTENANT DES TENSIO-ACTIFS, AUTO-CHAUFFANT, COMPRENANT UNE COMBINAISON DE PEG DE BAS POIDS MOLÉCULAIRE ET DE HAUT POIDS MOLÉCULAIRE

(30) Priorität: 04.02.2011 DE 102011003638
(43) Veröffentlichungstag der Anmeldung: 11.12.2013
(73) Patentinhaber: Beiersdorf AG, 20253 Hamburg (DE)
(72) Erfinder: HENTREY, Stefanie, 20259 Hamburg (DE); NEUHOFF, Henrike, 22359 Hamburg (DE); ECKERT, Julia, 20249 Hamburg (DE); RATHSACK, Jessica, 21614 Buxtehude (DE)
(74) Vertreter: Wihstutz, Kolja
(86) Internationale Anmeldenummer: PCT/EP2012/051000
(87) Internationale Veröffentlichungsnummer: WO 2012/104164

(56) Entgegenhaltungen:
- WO-A1-01/08654
- WO-A1-2006/118939
- DE-A1- 10 234 257
- DE-A1- 10 253 304
- DE-A1-102004 038 771
- FR-A1- 2 913 334
- GB-A- 1 317 771
- US-A1- 2008 253 973
- Anonymous: "Polyethylene Glycols", , 27 June 2002 (2002-06-27), XP055292161, Retrieved from the Internet: URL:http://www.fao.org/ag/agn/jecfa-additi ves/specs/Monograph1/Additive-316.pdf [retrieved on 2016-07-29]
- Anonymous: "INCI -Nomenclature Conventions", , 17 March 2008 (2008-03-17), XP055292168, Retrieved from the Internet: URL:http://www.surfatech.com/pdfs/INCI_Nam es.pdf [retrieved on 2016-07-29]

## Beschreibung

Die Erfindung betrifft kosmetische Reinigungsgele. Diese Produkte dienen dazu, die Haut zu reinigen. Sie sind gegenüber Seifen hautfreundlicher. Gegenüber Flüssigseifen, in denen häufig unverträgliche Tenside, wie Sodium Laureth Sulfat o.ä. eingesetzt werden, haben diese Reinigungsmittel den Vorteil, dass milde Tenside, z. B. Laureth-4 + MIPA Laureth Sulfate verwendet werden können. Dadurch sind diese Reinigungsformulierungen für die Gesichtsreinigung besonders geeignet. Diese Produkte sind so beschaffen, dass sie beim Lösen in Wasser Hydratationswärme freisetzen. Diese Wärme vermittelt bei der Anwendung ein sensorisch angenehmes Gefühl und unterstützt den Eindruck einer besonders effektiven Reinigung.

Alle Mengenangaben sind - sofern nicht anders angegeben - Massenprozente bezogen auf die Gesamtmasse der Zubereitung.

PEG im Sinne der vorliegenden Schrift sind Polyethylenglycolether.

"Selbstwärmend" im Sinne der vorliegenden Schrift bedeutet, dass sich das Produkt beim Auftragen auf die nasse Gesichtshaut fühlbar erwärmt, beispielsweise um durchschnittlich 7 bis 8 °C. Grundlage dafür ist ein flüssiger Träger, der bei Wasserzugabe solvatisiert wird und im Verlauf dieser exothermen Reaktion die negative Lösungsenthalpie in Form von Wärme an die Umgebung, in diesem Fall die Epidermis, abgibt.

Aus der FR 2913334 B1 war eine trockene Zubereitung mit einem exothermen Metallsalz, einer hydrophilen Kieselsäure und einer hydrophoben Kieselsäure bekannt.

Aus der FR 2930435 A1 war eine wasserfreie Zubereitung mit einem exothermen Metallsalz sowie einer hydrophilen Kieselsäure und einer hydrophoben Kieselsäure in bestimmten Verhältnissen bekannt.

Aus der EP 1877026 A1 war eine Zubereitung mit hydrophob modifizierter Kieselsäure, Partikeln und wenigstens 70 % wasserlöslicher Polyole mit einem Löslichkeitsparameter von 11 bis 17 bekannt.

Bei den bisher bekannten Reinigungsgelen wird die Hydratationswärme über Zeolithe oder Salze mit negativer Lösungsenthalpie erzeugt. Zeolithe haben jedoch den Nachteil, dass das Produkt einen stark alkalischen pH Wert (9 - 11) hat und bei Salzen tritt bei der Produktanwendung eine starke, osmotisch bedingte, Austrocknung der Epidermis auf. Das Potenzial von niedermolekularen PEGs ist ebenfalls bekannt, lässt sich jedoch nicht nutzen, da sich die dafür notwendigen 50 % kaum verdicken lassen. Gleichzeitig lassen sich diese Systeme, insbesondere in Kombination mit Tensiden, nur sehr begrenzt stabil formulieren. Es fehlte dem Stand der Technik an Zubereitungen, die einen cremigen Schaum entwickeln, sich leicht abwaschen lassen und bei der Anwendung einen angenehmen Wärmeeffekt haben. Die Intensität der Wärme sollte dabei als gerade richtig, d. h. weder als zu stark, noch als zu schwach, wahrgenommen werden. Zugleich war es wünschenswert, dass solche Zubereitungen die Gesichtshaut besonders gründlich und dabei auf sanfte Weise reinigen und dabei nach der Anwendung ein angenehmes Hautgefühl hinterlassen.

Der Fachmann kennt auch das Dokument DE 10234257 A1, welches kosmetische Reinigungszubereitungen enthaltend niedermolekulare Polxethylenglykole als Tränkungsmedium für Tücher offenbart. Die Zubereitungen sind ebenfalls selbstwärmend, enthalten jedoch keine Polyethylenglykole mit 20 bis 180 Ethylenoxideinheiten in einem Anteil von mindestens 9 Gew.-%.

Des Weiteren kennt der Fachmann das Dokument DE102004038771 A1, welches ebenfalls selbstwärmende Hautreinigungszusammensetzungen offenbart. Jedoch konnte auch dieses Dokument keinen Hinweis auf die vorliegende Erfindung liefern.

Überraschend und für den Fachmann nicht vorhersehbar, hat sich nun herausgestellt, dass ein selbsterwärmendes kosmetisches Reinigungsgel mit einem Gehalt an Tensiden enthaltend a) mehr als 50 Gew.-% wassermischbaren Hydroxylverbindungen, ausgewählt aus Polyethylenglycolether mit 6 bis 10 Ethylenoxideinheiten, wobei diese Hydroxylverbindungen bei 25°C flüssig sind, unter der Maßgabe, dass die gewählten Substanzen beim Lösen in Wasser Wärme entwickeln, also eine negative Lösungsenthalpie besitzen, b) mindestens 9 Gew.-% hochmolekulares PEG mit 20 bis 180 Ethylenoxideinheiten den Mängeln des Standes der Technik abhilft. Dadurch bildet sich eine Gelstruktur aus, die andere Inhaltstoffe stabilisiert. Diese werden beim Mischen mit Wasser solvatisiert und geben dabei die negative Lösungsenthalpie in Form von Wärme ab. Diese liegen bei Raumtemperatur als Feststoff vor. Besonders bevorzugt ist es, wenn das Verhältnis zwischen den hochmolekularen PEGs und den niedermolekularen PEGs zwischen 1:7 und 1:12 liegt, ganz besonders bevorzugt 1:9 ist. Dann liegt ausreichend hochmolekulares PEG vor, das eine Gelstruktur ausbilden kann, in die das niedermolekulare PEG im Herstellungsverlauf eingelagert und stabilisiert wird. Dadurch werden die maximale Hydratationsenthalpie und die Viskosität der Formulierung vorteilhaft beeinflusst. Weiter ist es bevorzugt, wenn zusätzlich eine mit Dimethyl Silylaten hydrophobisierte Kieselsäure enthalten ist, bevorzugt mit einem Gehalt von 0,1 bis 6,0 Gew.-%, besonders bevorzugt 2,5 bis 3,5 Gew.-%. Ein Beispiel für diese Kieselsäure ist das unter dem Handelsnamen Aerosil 972 erhältliche Silica Dimethyl Silylat von der Firma Degussa. Diese Rohstoffgruppe ist dadurch gekennzeichnet, dass die Adhesivität der Formel bei der Produktanwendung auf der Haut verringert und dadurch ein sensorisch angenehmes Hautgefühl vermittelt. Parallel dazu werden die Silica Dimethyl Silylat Partikel in die Gelstruktur der hochmolekularen PEGs eingelagert und verbessern die Lagerstabilität der Formel. Bevorzugt ist es, wenn 0,5 Gew.-% bis 15 Gew.-% an anionischen, zwitterionischen, amphoteren oder nichtionischen Tensiden enthalten sind, besonders bevorzugt 4,5 Gew.-% bis 10,5 Gew.-% einer Mischung von zwei oder mehr unterschiedlichen Tensidtypen. Besonders bevorzugt sind die Tenside wasserfrei sind bzw. weisen einen Wassergehalt < 1 % auf. Dadurch entwickelt sich beim Aufschäumen während der Produktanwendung ein cremiger Schaum und die Produkte sind besonders mild. Weiter bevorzugt ist es, wenn der Wassergehalt höchstens 0,9 Gew.-%, besonders bevorzugt höchstens 0,1 Gew.-% beträgt. Dadurch wird erreicht, dass die niedermolekularen PEG Moleküle in der Zubereitung im dehydratisierten Zustand vorliegen und erst bei dem Wasserkontakt während der Produktanwendung solvatisiert werden. Besonders bevorzugt ist es, wenn 0,01 Gew.-% bis 15 Gew.-% Glyceryl Glucose (Hydagen GG, zusammensetzt aus 54 % Glycerin, 43 % Glyceryl Glucoside und 3 % Wasser, Firma Cognis) enthalten sind.

Vorteilhaft können solche Zubereitungen zusätzlich mind. 0,01 % und maximal 15 % Jeesperse CPW-P, ein Compound aus Cetearyl Alcohol, Sodium Polyacrylate und Polysorbate-60; mind. 0,01 % und maximal 15 % Jeesperse CPW-5, ein Compound aus Polyethylene und Sodium Polyacrylate oder mind. 0,01 % und maximal 10 % Jeenate 3H, ein Compound aus Ethylene Homopolymer enthalten.

### Beispiele

Folgende Produkte wurden hergestellt und in einer Probandenstudie mit n = 160 Probanden miteinander verglichen:
alle Konzentrationen in Gew.-%

| **INCI** | **Produkt A** (erfinderisch) | **Produkt B** (Vergleichsprodukt) |
|---|---|---|
| Magnolia Officinalis Bark Extract | 0.05 | 0.05 |
| PEG-150 | 9.00 | |
| PEG-40 Hydrogenated Castor Oil | 0.70 | 0.30 |
| PEG-7 Glyceryl Cocoate | 0.55 | |
| Silica | | 1.40 |
| Talc | | 1.20 |
| Silica Dimethyl Silylate | 3.50 | 4.35 |
| Parfum | 0.60 | 0.60 |
| Glycerin | 13.60 | 26.41 |
| Propylene Glycol | | 32.78 |
| Magnesium Sulfate | | 12.00 |
| PEG-8 | 62.00 | 16.71 |
| Laureth-4 + MIPA Laureth Sulfate + PEG-6 Caprylic/Capric Glycerides + Propylene Glycol | 10.00 | 4.20 |

### Ergebnisse

Die Produkt A ist bei 32 °C für 6 Monate lagerstabil. Das entspricht in der Regel einer Mindesthaltbarkeit im Regal des Einzelhandels von 24 Monaten.

Die sensorische Beurteilung der Probanden erfolgte auf einer Skala von 1 (trifft überhaupt nicht zu) bis 7 (trifft voll und ganz zu).

| **Kriterium** | **Produkt A** (erfinderisch) | **Produkt B** (Vergleichs-produkt) |
|---|---|---|
| Cremiger Schaum | 5,6 | 5,3 |
| Leichte Abwaschbarkeit | 6,0 | 5,9 |
| Angenehmer Wärmeeffekt | 6,1 | 6,1 |
| Unterstützung der Effektivität des Produktes durch den Wärmeeffekt | 6,1 | 6,1 |
| Gründliche Reinigung der Gesichtshaut | 6,1 | 5,8 |
| Sanfte Reinigung der Gesichtshaut | 6,1 | 5,9 |
| Angenehmes Hautgefühl nach der Anwendung | 6,1 | 5,8 |

Die Probanden beurteilten das erfinderische Produkt A insgesamt besser als das Vergleichsprodukt B.

Desweiteren wurde separat abgefragt, ob die Intensität des Wärmeeffektes als genau richtig empfunden wird.

| **Kriterium** | **Produkt A** (erfinderisch) | **Produkt B** (Vergleichsprodukt) |
|---|---|---|
| Die Intensität des Wärmeeffektes ist genau richtig. | 74% | 71 % |

Auch hinsichtlich des wichtigen Merkmals der richtigen Wärmeentwicklung ist das erfinderische Produkt A dem Vergleichsprodukt B überlegen.

### Weitere Beispielrezepturen:

### Rezeptur 1:

| **INCI Bezeichnung** | **Anteil [Gew.-%]** |
|---|---|
| Magnolia Officinalis Bark Extract | 0.0500 |
| PEG-150 | 7.5000 |
| PEG-7 Glyceryl Cocoate | 0.5500 |
| PEG-40 Hydrogenated Castor Oil | 0.6000 |
| Silica Dimethyl Silylate | 4.0000 |
| Parfum | 0.6000 |
| Glycerin | 14.7000 |
| PEG-8 | 67.0000 |
| Laureth-4 + MIPA Laureth Sulfate + PEG-6 Caprylic/Capric Glycerides + Propylene Glycol | 5.0000 |

### Rezeptur 2 - nicht teil der Erfindung:

| **INCI Bezeichnung** | **Handelsname (Hersteller)** | **Anteil [Gew.-%]** |
|---|---|---|
| PEG-20 | Lipoxol 1000 MED (Sasol), PEG-1000 (Clariant) | 2.0000 |
| PEG-32 | Lipoxol 1500 MED (Sasol) | 3.0000 |
| PEG-60 | Lipoxol 3000 MED (Sasol) | 3.0000 |
| PEG-150 | Lipoxol 6000 MED (Sasol) | 4.0000 |
| PEG-7 Glyceryl Cocoate | Cetiol HE (Cognis), Glycerox HE B (Croda) | 1.1000 |
| PEG-40 Hydrogenated Castor Oil | Cremophor CO 40 (BASF), Eumulgin HRE 40 (Cognis) | 0.7000 |
| Silica Dimethyl Silylate | Aerosil R972 (Reher & Ramsden) | 2.0000 |
| Parfum | | 0.6000 |
| Glycerin | Refined Glycerine 99,5% (Peter Cremer), Glycerol EP vegetable 99,5% (Spiga Nord) | 13.6000 |
| PEG-12 | POLYGLYCOL 600 (Clariant) | 20.0000 |
| PEG-8 | POLYGLYCOL 400 (Clariant) | 40.0000 |
| Laureth-4 + MIPA Laureth Sulfate + PEG-6 Caprylic/Capric Glycerides + Propylene Glycol | Softigen M 40 (Sasol) | 10.0000 |

### Rezeptur 3 - nicht Teil der Erfindung:

| **INCI Bezeichnung** | **Handelsname (Hersteller)** | **Anteil [Gew.-%]** |
|---|---|---|
| Magnolia Officinalis Bark Extract | Magnolia Bark Extract (Layn Natural Ingredients) | 0.0500 |
| PEG-150 | Lipoxol 6000 MED (Sasol) | 11.0000 |
| PEG-7 Glyceryl Cocoate | Cetiol HE (Cognis), Glycerox HE B (Croda) | 0.5500 |
| PEG-40 Hydrogenated Castor Oil | Cremophor CO 40 (BASF), Eumulgin HRE 40 (Cognis) | 0.7000 |
| Silica Dimethyl Silylate | Aerosil R972 (Reher & Ramsden ) | 3.5000 |
| Parfum | | 0.6000 |
| Glycerin | Refined Glycerine 99,5% (Peter Cremer), Glycerol EP vegetable 99,5% (Spiga Nord ) | 27.6000 |
| PEG-8 | Polyglykol 400 (Clariant) | 50.0000 |
| Laureth-4 + MIPA Laureth Sulfate + PEG-6 Caprylic/Capric Glycerides + Propylene Glycol | Softigen M 40 (Sasol) | 10.0000 |

### Herstellungsprozess

1. Zu Beginn Glycerin, PEG-150 und die Hälfte der PEG-8 Menge auf 75 °C aufheizen und die Temperatur konstant halten bis zur vollständigen Schmelzung des PEG- 150.
2. Die verbleibende PEG 8 Menge auf 70 °C erwärmen und das Silica Dimethyl Silylat einrühren.
3. Beide Phasen miteinander verrühren.
4. Anschließend wird der Kühlprozess initiiert. Mit Hilfe des Ramp Time Cooling den Ansatz in kleinen Temperaturschritten auf 35°C abkühlen. Insbesondere zwischen 60 und 45 °C müssen die Zielpunkte der Kühlschritte sehr nah beieinander liegen, da in diesem Bereich der Erstarrungspunkt des PEG-150 liegt. Bei diesem Kühlprozess ist der Mantel mit "warmem" Wasser gefüllt. Die Schwierigkeit ist dabei, dass das PEG-150 als homogenes Netzwerk auskristallisiert und nicht als Schicht an der kälteren Manteloberfläche erstarrt. Dies wird durch eine exakte Führung des Kühlgradienten bei einer max. Temperaturdifferenz von 2 - 5 °C zwischen Füllgut und Manteloberfläche erreicht.
5. Generell sollten unterhalb einer Temperatur von 35 °C keine sehr starken Scherkräfte angewendet werden, da dadurch Teile des PEG Gerüsts irreversibel zerstört.
6. Wenn der Ansatz eine Temperatur von 40 °C hat, kann die Tensid-Phase zugegeben werden.
7. Wenn die Phase homogen im Ansatz verteilt ist, kann die Parfümphase zugegeben werden.
8. Am Ende des Herstellungsprozesses sollte der Ansatz ca. einmal im Umlauf gepumpt werden. Anschließend wird der Ansatz bei laufendem Homogenisator ausgetragen.
9. Die Zubereitungen 1 bis 3 sind bei 32 °C für 6 Monate lagerstabil. Das entspricht in der Regel einer Mindesthaltbarkeit im Regal des Einzelhandels von 24 Monaten.

## Patentansprüche

1. Selbsterwärmendes kosmetisches Reinigungsgel mit einem Gehalt an Tensiden enthaltend
a) mehr als 50 Gew.-% wassermischbare Hydroxylverbindungen, ausgewählt aus Polyethylenglycolether mit 6 bis 10 Ethylenoxideinheiten, wobei diese Hydroxylverbindungen bei 25°C flüssig sind, unter der Maßgabe, dass die gewählten Substanzen beim Lösen in Wasser Wärme entwickeln,
b) mindestens 9 Gew.-% hochmolekulares PEG mit 20 bis 180 Ethylenoxyeinheiten.

2. Zubereitung nach Patentanspruch 1, **dadurch gekennzeichnet, dass** Verhältnis zwischen den hochmolekularen PEGs und den niedermolekularen PEGs zwischen 1:7 und 1:12 liegt, besonders bevorzugt 1:9 ist.

3. Zubereitung nach einem der vorangehenden Patentansprüche, **dadurch gekennzeichnet, dass** zusätzlich eine mit Dimethyl Silylaten hydrophobisierte Kieselsäure enthalten ist, bevorzugt mit einem Gehalt von 0,1 bis 6,0 Gew.-%, besonders bevorzugt 2,5 bis 3,5 Gew.-%.

4. Zubereitung nach einem der vorangehendenden Patentansprüche, **dadurch gekennzeichnet, dass** 0,5 Gew.-% bis 15 Gew.-% an anionischen, zwitterionischen, amphoteren oder nichtionischen Tensiden enthalten sind, besonders bevorzugt 4,5 Gew.-% bis 10,5 Gew.-% einer Mischung von zwei oder mehr unterschiedlichen Tensidtypen.

5. Zubereitung nach einem der vorangehendenden Patentansprüche, **dadurch gekennzeichnet, dass** der Wassergehalt höchstens 0,9 Gew.-%, besonders bevorzugt höchstens 0,1 Gew.-% beträgt.

## Claims

1. Self-heating cosmetic cleansing gel having a content of surfactants comprising
a) more than 50 wt% water-miscible hydroxyl compounds selected from polyethylene glycol ethers having 6 to 10 ethylene oxide units, wherein said hydroxyl compounds are liquid at 25°C, with the proviso that the selected substances develop heat on dissolution in water,
b) at least 9 wt% high molecular weight PEG having 20 to 180 ethyleneoxy units.

2. Preparation according to Patent Claim 1, **characterized in that** the ratio between the high molecular weight PEGs and the low molecular weight PEGs is between 1:7 and 1:12, and is particularly preferably 1:9.

3. Preparation according to either of the preceding patent claims, **characterized in that** a silica hydrophobized with dimethyl silylates is also present, preferably with a content of 0.1 to 6.0 wt%, particularly preferably 2.5 to 3.5 wt%.

4. Preparation according to any of the preceding patent claims, **characterized in that** 0.5 wt% to 15 wt% of anionic, zwitterionic, amphoteric or nonionic surfactants are present, particularly preferably 4.5 wt% to 10.5 wt% of a mixture of two or more different surfactant types.

5. Preparation according to any of the preceding patent claims, **characterized in that** the water content is at most 0.9 wt%, particularly preferably at most 0.1 wt%.

## Revendications

1. Gel nettoyant cosmétique auto-chauffant ayant une teneur en tensioactifs, contenant :
a) plus de 50 % en poids de composés hydroxyle miscibles avec l'eau, choisis parmi les éthers de polyéthylène glycol contenant 6 à 10 unités oxyde d'éthylène, ces composés hydroxyle étant liquides à 25 °C, à condition que les substances choisies libèrent de la chaleur lors de la dissolution dans l'eau,
b) au moins 9 % en poids de PEG de poids moléculaire élevé contenant 20 à 180 unités éthylène-oxy.

2. Préparation selon la revendication 1, **caractérisée en ce que** le rapport entre les PEG de poids moléculaire élevé et les PEG de faible poids moléculaire est compris entre 1:7 et 1:12, de manière particulièrement préférée est de 1:9.

3. Préparation selon l'une quelconque des revendications précédentes, **caractérisée en ce qu'**une silice hydrophobée avec des silylates de diméthyle est en outre contenue, de préférence en une teneur de 0,1 à 6,0 % en poids, de manière particulièrement préférée de 2,5 à 3,5 % en poids.

4. Préparation selon l'une quelconque des revendications précédentes, **caractérisée en ce que** 0,5 % en poids à 15 % en poids de tensioactifs anioniques, zwitterioniques, amphotères ou non ioniques sont contenus, de manière particulièrement préférée 4,5 % en poids à 10,5 % en poids d'un mélange de deux types de tensioactifs différents ou plus.

5. Préparation selon l'une quelconque des revendications précédentes, **caractérisée en ce que** la teneur en eau est d'au plus 0,9 % en poids, de manière particulièrement préférée d'au plus 0,1 % en poids.
